# EUROPÄISCHE PATENTSCHRIFT

(11) **EP 0 392 286 B1**
(45) Veröffentlichungstag und Bekanntmachung des Hinweises auf die Patenterteilung: **18.11.1993**
(21) Anmeldenummer: 90106113.5
(22) Anmeldetag: 30.03.1990
(51) Int. Cl.: C07D 207/34, A01N 43/36, C07C 255/35, C07C 255/37

(54) **3-Cyano-4-phenyl-pyrrol-Derivate**
3-Cyano-4-phenyl-pyrrole derivatives
Dérivés de 3-cyano-4-phényl-pyrrole

(30) Priorität: 13.04.1989 DE 3912156
(43) Veröffentlichungstag der Anmeldung: 17.10.1990
(73) Patentinhaber: BAYER AG, 51368 Leverkusen (DE)
(72) Erfinder: Wollweber, Detlef, Dr., D-5600 Wuppertal 1 (DE); Brandes, Wilhelm, Dr., D-5653 Leichlingen 1 (DE); Dutzmann, Stefan, Dr., D-4000 Düsseldorf (DE); Hänssler, Gerd, Dr., D-5090 Leverkusen 3 (DE)

(56) Entgegenhaltungen:
- EP-A- 0 182 738
- EP-A- 0 277 537
- EP-A- 0 281 731
- EP-A- 0 315 869
- EP-A- 0 318 704
- DE-A- 2 927 480

## Beschreibung

Die Erfindung betrifft neue 3-Cyano-4-phenyl-pyrrol-Derivate, ein Verfahren zu ihrer Herstellung, ihre Verwendung als Fungizide, sowie neue Zwischenprodukte.

Es ist bekannt, daß bestimmte 3-Cyano-4-phenyl-pyrrole, wie beispielsweise die Verbindung 3-Cyano-4-(2,3-dichlorphenyl)-pyrrol, fungizide Wirksamkeit besitzen (vgl. z.B. EP-A 0 236 272).

Weiterhin ist bereits bekannt, daß bestimmte Perhalogenalkylmercapto-sulfonamide und -sulfamide, wie beispielsweise N,N-Dimethyl-N'-phenyl-N'-(fluordichlormethylthio)-sulfamid, fungizide Eigenschaften aufweisen (vgl. DA-B 1 193 498).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Aus den vorgängigen, aber nicht vorveröffentlichten Schutzrechten gemäß EP-A 0 315 869 und EP-A 0 318 704 sind ebenfalls 3-Cyano-4-phenyl-pyrrole mit fungiziden Eigenschaften bekannt. Es werden aber keine Verbindungen dieses Typs offenbart, in denen der Phenylrest in 2-und/oder 3-Stellung durch eine Halogenalkylgruppe substituiert ist und außerdem noch einen oder zwei weitere Reste enthält, von denen aber höchstens einer für Fluor steht und dieser weder in 2- noch in 3-Position gebunden ist.

Aus der EP-A 0 277 537 geht hervor, daß 3-Cyano-4-phenylpyrrole als Ausgangsstoffe zur Herstellung von N-substituierten Pyrrol-Derivaten eingesetzt werden können. Es werden aber nur solche Zwischenprodukte erwähnt, in denen der Phenylrest in 2- und 3-Position durch Chlor substituiert ist.

Es wurden neue 3-Cyano-4-phenyl-pyrrol-Derivate der Formel (I), 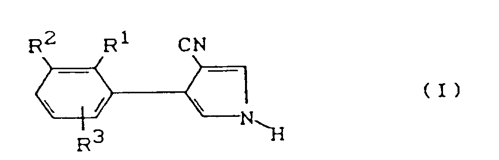
in welcher
- R¹: für Chlor, Brom, geradkettiges oder verzweigtes Alkyl mit 1 bis 4 Kohlenstoffatomen oder für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen steht,
- R²: für Chlor, Brom, geradkettiges oder verzweigtes Alkyl mit 1 bis 4 Kohlenstoffatomen oder für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen steht und
- R³: für Wasserstoff oder Fluor steht,
mit der Maßgabe, daß mindestens einer der Reste R¹ oder R² für Halogenalkyl steht;
gefunden.

Weiterhin wurde gefunden, daß man die neuen 3-Cyano-4-phenyl-pyrrole der Formel (I)
erhält, wenn man substituierte Zimtsäurenitrile der Formel (II) 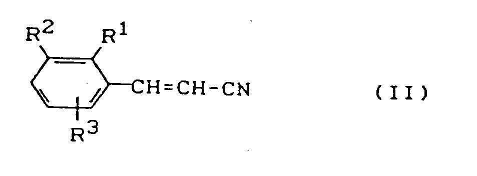
in welcher
- R¹, R² und R³: die oben angegebene Bedeutung haben,
mit Sulfonylmethylisocyaniden der Formel (III),

R⁴-SO₂-CH₂-NC (III)

in welcher
- R⁴: für Alkyl mit 1 bis 4 Kohlenstoffatomen
oder für gegebenenfalls einfach substituiertes Phenyl steht in Gegenwart einer Base und gegebenenfalls in Gegenwart eines Verdünnungsmittels umsetzt.

Schließlich wurde gefunden, daß die neuen 3-Cyano-4-phenyl-pyrrole der allgemeinen Formel (I) eine gute Wirkung gegen Pilze besitzen.

Überraschenderweise zeigen die erfindungsgemäßen 3-Cyano-4-phenyl-pyrrole der allgemeinen Formel (I) eine erheblich bessere fungizide Wirksamkeit als z.B. die aus dem Stand der Technik bekannten 3-Cyano-4-phenyl-pyrrole und Perhalogenalkylmercapto-sulfonamide und -sulfamide, wie das 3-Cyano-4-(2,3-dichlor-phenyl)-pyrrol und das N,N-Dimethyl-N'-phenyl-N'-(fluordichlormethylthio)-sulfamid, welche chemisch und wirkungsmäßig naheliegende Verbindungen sind.

Die erfindungsgemäßen 3-Cyano-4-phenyl-pyrrole sind durch die Formel (I) allgemein definiert. Bevorzugt sind Verbindungen der Formel (I), bei welchen
- R¹: für Chlor, Brom, Methyl, Ethyl, Trifluormethyl oder Trifluormethoxy steht,
- R²: für Chlor, Brom, Methyl, Ethyl, Trifluormethyl oder Trifluormethoxy steht und
- R³: für Wasserstoff oder Fluor steht,
mit der Maßgabe, daß mindestens einer der Reste R¹ oder R² für Trifluormethyl steht.

Als ganz besonders bevorzugte Verbindungen der Formel (I) seien genannt:
3-Cyano-4-(2-methyl-3-trifluormethylphenyl)-pyrrol und
3-Cyano-4-(2,3-Di-trifluormethylphenyl)pyrrol.

Die für die Verbindungen der Formel (I) angegebenen bevorzugten Definitionen gelten auch für die Ausgangsverbindungen der Formel (II).

Im einzelnen seien außer den bei den Herstellungsbeispielen genannten Verbindungen die folgenden 3-Cyano-4-phenyl-pyrrole der allgemeinen Formel (I) genannt:

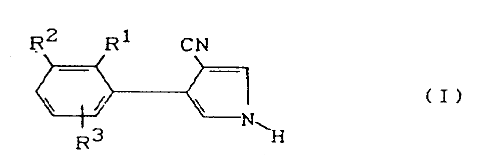

**Tabelle 1**

| R¹ | R² | R³ |
|---|---|---|
| CF₃ | Cl | H |
| CF₃ | CH₃ | H |
| CF₃ | OCF₃ | H |
| CF₃ | Br | H |
| Cl | CF₃ | H |
| Br | CF₃ | H |
| OCF₃ | CF₃ | H |
| CF₃ | Cl | 4F |
| CF₃ | Cl | 5F |
| CF₃ | CH₃ | 4F |
| CF₃ | CH₃ | 5F |
| CF₃ | OCF₃ | 4F |
| CF₃ | OCF₃ | 5F |
| Cl | CF₃ | 4F |
| Cl | CF₃ | 5F |
| Br | CF₃ | 4F |
| Br | CF₃ | 5F |
| OCF₃ | CF₃ | 4F |
| OCF₃ | CF₃ | 5F |
| CH₃ | CF₃ | 4F |
| CH₃ | CF₃ | 5F |

Verwendet man beispielsweise 2-Methyl-3-trifluormethylzimtsäurenitril und p-Toluolsulfonylmethylisocyanid als Ausgangsstoffe und Natriumhydrid als Base, so läßt sich der Reaktionsablauf des erfindungsgemäßen Verfahrens durch das folgende Formelschema darstellen: 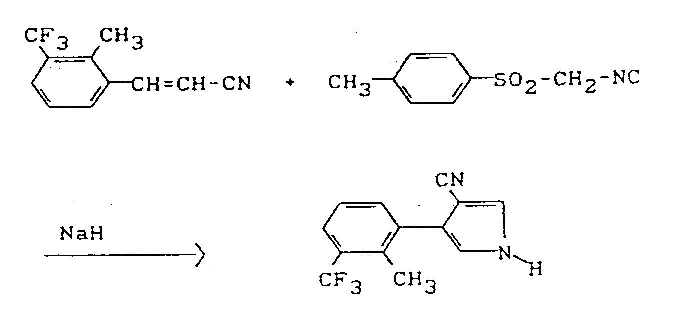
Die zur Durchführung des erfindungsgemäßen Verfahrens als Ausgangsstoffe benötigten substituierten Zimtsäurenitrile sind durch die Formel (II) allgemein definiert. In dieser Formel (II) stehen R¹, R² und R³ vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Zimtsäurenitrile der Formel (II) sind neu. Man erhält sie jedoch in Analogie zu bekannten verfahren (vgl. z.B. DE-A 2 927 480), beispielsweise wenn man
a) Aniline der Formel (IV), 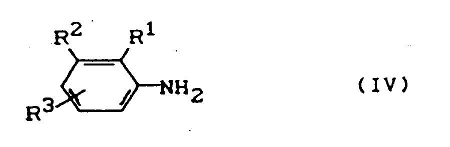 in welcher
   R¹, R² und R³ die oben angegebene Bedeutung haben, mit der Maßgabe, daß mindestens einer der Reste R¹ oder R² für Halogenalkyl steht,
   zunächst in einer ersten Stufe mit Acrylnitril unter üblichen Diazotierungsbedingungen, beispielsweise in Gegenwart von Natriumnitrit und Salzsäure, und in Gegenwart eines geeigneten Metallsalz-Katalysators, wie beispielsweise Kupfer-II-chlorid oder Kupfer-II-oxid, und gegebenenfalls in Gegenwart eines geeigneten Verdünnungsmittels, wie beispielsweise Aceton oder Wasser, bei Temperaturen zwischen - 20 °C und 50 °C umsetzt ("Meerwein-Arylierung"; vgl. hierzu auch Organic Reactions 11, 189 [1960]; Organic Reactions 24, 225 [1976] oder C. Ferri "Reaktionen der organischen Synthese" S. 319, Thieme Verlag Stuttgart 1978) und dann in einer 2. Stufe die so erhältlichen substituierten α-Chlor-β-phenylpropionitrile der Formel (V), 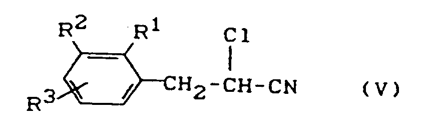 in welcher
   R¹, R² und R³ die oben angegebene Bedeutung haben,
   mit der Maßgabe, daß mindestens einer der Reste R¹ oder R² für Halogenalkyl steht
   mit Basen, wie beispielsweise Triethylamin oder Diazabicycloundecen, in üblicher Art und Weise gegebenenfalls in Gegenwart eines Verdünnungsmittels, wie beispielsweise Tetrahydrofuran, bei Temperaturen zwischen 0 °C und 50 °C dehydrohalogeniert (vgl. auch die Herstellungsbeispiele) oder alternativ, wenn man
b) Benzaldehyde der Formel (VI), 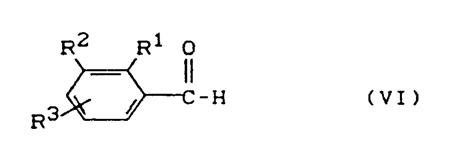 in welcher
   R¹, R² und R³ die oben angegebene Bedeutung haben,
   mit der Maßgabe, daß mindestens einer der Reste R¹ oder R² für Halogenalkyl steht.
mit Cyanessigsäure der Formel (VII),

NC-CH₂-COOH (VII)

in üblicher Art und Weise in Gegenwart einer Base, wie beispielsweise Piperidin oder Pyridin, und gegebenenfalls in Gegenwart eines geeigneten Verdünnungsmittels, wie beispielsweise Pyridin, bei Temperaturen zwischen 50 °C und 120 °C kondensiert und gleichzeitig decarboxyliert (vgl. z.B. "Organikum" S. 571/572; 15. Auflage; VEB Deutscher Verlag der Wissenschaften Berlin 1981 sowie die Herstellungsbeispiele).

Die Aniline der Formel (IV) sind teilweise bekannt (vgl. z.B. DE-A 3726891, JP-A 62298562, EP-A 206951, JP-A 61083146) oder erhältlich in Analogie zu bekannten Verfahren.

Die weiterhin als Vorprodukte zur Herstellung der neuen Ausgangsprodukte der Formel (II) nach Variante b) benötigten Benzaldehyde der Formel (VI) sind größtenteils bekannt (vgl. z. B. EP-A 225 175, EP-A 125 803, EP-A 174 131, EP-A 169 009, EP-A 168 151, EP-A 145 334, US-A 4 572 909, JP-A 84 118 782, J. Chem. Soc., Perkin Trans. 1, 1837-1844 und J. Med. Chem. 29, 1696-1702). Die Cyanessigsäure der Formel (VII) ist ebenfalls eine allgemein bekannte Verbindung der organischen Chemie.

Die zur Durchführung des erfindungsgemäßen Verfahrens weiterhin als Ausgangsstoffe benötigten Sulfonylmethylisocyanide sind durch die Formel (III) allgemein definiert. In dieser Formel (III) steht R⁴ vorzugsweise für 4-Methylphenyl, 4-Chlorphenyl oder für Phenyl.

Die Sulfonylmethylisocyanide der Formel (III) sind bekannt (vgl. z.B., Synthesis 1985, 400-402; Org. Syntheses 57, 102-106 [1977]; J. org. Chem. 42, 1153-1159 [1977]; Tetrahedron Lett. 1972, 2367-2368).

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Amide, wie Dimethylformamid, Dimethylacetamid, N-Methylformanilid, N-Methylpyrrolidon oder Hexamethylphosphorsäuretriamid, oder Sulfoxide, wie Dimethylsulfoxid.

Das erfindungsgemäße Verfahren kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-C₁₃/C₁₅-alkylammoniumchlorid, Dibenzyl-dimethyl-ammoniummethylsulfat, Dimethyl-C₁₂/C₁₄-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Das erfindungsgemäße Verfahren wird vorzugsweise in Gegenwart einer geeigneten Base durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen - 30 °C und + 120 °C, vorzugsweise bei Temperaturen zwischen - 20 °C und + 50 °C.

Zur Durchführung des erfindungsgemäßen Verfahrens setzt man pro Mol an substituiertem Zimtsäurenitril der Formel (II) im allgemeinen 1.0 bis 2.0 Mol, vorzugsweise 1.0 bis 1.5 Mol an Sulfonylmethylisocyanid der Formel (III) und 1.0 bis 2.0 Mol, vorzugsweise 1.0 bis 1.5 Mol an Base ein.

Dabei ist es gegebenenfalls von Vorteil, die Reaktion in Gegenwart einer Schutzgasatmosphäre wie beispielsweise Argon durchzuführen.
Die Reaktionsdurchführung, Aufarbeitung und Isolierung der Reaktionsprodukte erfolgt nach allgemein üblichen Methoden.

Alternativ zur Herstellung der erfindungsgemäßen Wirkstoffe mit Hilfe des erfindungsgemäßen Herstellungsverfahrens sind verschiedene weitere Herstellungsverfahren zur Herstellung der erfindungsgemäßen Wirkstoffe denkbar.

So erhält man erfindungsgemäße Wirkstoffe der Formel (I) beispielsweise auch, wenn man α-Cyanozimtsäureester in Gegenwart von Basen und in Gegenwart von Kupfer-(II)-Salzen mit p-Toluolsulfonylmethylisocyanid umsetzt (vgl. J6-1030-571 oder J6-1200-984) oder wenn man α-substituierte Zimtsäurenitrile in Gegenwart von Natriumhydrid mit Isocyanoessigsäureestern cyclisiert, die so erhältlichen Pyrrol-2-carbonsäureester mit Basen verseift und anschließend thermisch decarboxyliert (vgl. JP-A 59/212468) oder wenn man Phenacylaminderivate mit geeignet substituierten Acrylnitril-Derivaten umsetzt (vgl. EP-A 174 910) oder wenn man 3-Trifluormethyl-4-phenyl-pyrrole mit Ammoniak bei erhöhter Temperatur und erhöhtem Druck umsetzt (vgl. EP-A 182 738) oder wenn man 3-Cyano-4-phenyl-Δ²-pyrroline in Gegenwart von Kupfer-II-Salzen oder Eisen-III-salzen oxidiert (vgl. EP-A 183 217) oder wenn man α-Cyanoacrylsäurederivate mit Isocyanoessigsäureestern in Gegenwart einer Base umsetzt und die so erhältlichen Δ²-Pyrrolin-2-carbonsäurederivate in einer 2. Stufe in Gegenwart einer Base und in Gegenwart eines Metallsalzkatalysators oxidativ decarboxyliert (vgl. DE-A 3 718 375.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum; Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Obst- und Gemüseanbau, wie beispielsweise gegen den Erreger des Bohnengrauschimmels (Botrytis cinerea) oder zur Bekämpfung von Reiskrankheiten wie beispielsweise gegen den Erreger der Reisfleckenkrankheit (Pyricularia oryzae) oder gegen Erysiphe graminis an Gerste einsetzen.

Weiterhin zeigen einige der erfindungsgemäßen Wirkstoffe gute fungizide Wirkungen gegen Venturia an Apfel, Pellicularia an Reis, Leptosphaeria, Cochliobolus, Pyrenophora und Fusarium an Getreidekulturen. Ferner zeigen einige der erfindungsgemäßen Wirkstoffe auch eine gute in vitro-Wirkung.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

### Herstellungsbeispiele

### Beispiel 1

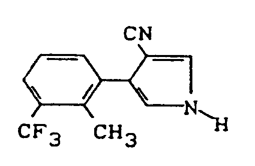

Zu 1,0 g (0.033 Mol) Natriumhydrid (80 %ig in Mineralöl) in 17,0 ml Tetrahydrofuran unter einer Argonschutzgasatmosphäre gibt man bei - 10 °C bis - 20 °C tropfenweise unter Rühren eine Lösung von 5,0 g (0.0237 Mol) 3-(2-Methyl-3-trifluormethyl-phenyl)-acrylnitril und 6,0 g (0.0308 Mol) p-Toluolsulfonylmethylisocyanid in 20 ml einer Mischung aus Tetrahydrofuran/Dimethylsulfoxid (6:1). Nach beendeter Zugabe läßt man die Reaktionsmischung auf Raumtemperatur kommen, gibt Wasser zu, extrahiert mehrfach mit Essigester, wäscht die vereinigten Essigesterphasen mit Wasser, trocknet über Natriumsulfat und engt im Vakuum ein. Der Rückstand wird durch Chromatographie an Kieselgel (Laufmittel: Cyclohexan/Essigester 5:1) gereinigt.

Man erhält 4,5 g (76 % der Theorie) an 3-Cyano-4-(2-methyl-3-trifluormethylphenyl)-pyrrol vom Schmelzpunkt 99 °C -100 °C.

### Beispiel 2

In analoger Weise zu Beispiel 1 und unter Berücksichtigung der Angaben in der Beschreibung des erfindungsgemäßen Verfahrens wird 3-Cyano-4-(2,3-Di-trifluormethyl-phenyl)-pyrrol vom Schmelzpunkt 147°C-149°C erhalten.

### Herstellung der Ausgangsverbindungen

### Beispiel II-1

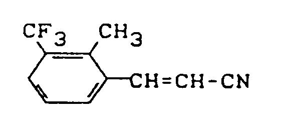

Zu 21,2 g (0.086 Mol) 2-Chlor-3-(2-methyl-3-trifluormethyl-phenyl)-propionitril in 120 ml Tetrahydrofuran tropft man bei Raumtemperatur unter Rühren 14,4 g (0,094 Mol) Diazabicycloundecen, rührt nach beendeter Zugabe 15 Stunden bei Raumtemperatur, filtriert, engt das Filtrat im Vakuum ein, nimmt den Rückstand in Essigester auf, wäscht nacheinander mit 1 normaler Salzsäure und Wasser, trocknet über Natriumsulfat und entfernt das Lösungsmittel im Vakuum.

Nach Destillation in einer Kugelrohrdestillationsapparatur erhält man 7,9 g (44% der Theorie) 3-(2-Methyl-3-trifluormethyl-phenyl)-acrylnitril vom Schmelzpunkt 87°C-88°C.

### Beispiel II-2

In analoger Weise zu Beispiel (II-1) und gemäß den allgemeinen Angaben zur Herstellung wird 3-(2,3-Trifluormethyl-phenyl)-acrylnitril erhalten. Die ¹H-NMR-Spektren (CDCl₃) δ = 5,7 - 5,9 1H (dd, dd)ppm wurden in Deuterochloroform (CDCl₃) mit Tetramethylsilan (TMS) als innerem Standard aufgenommen. Angegeben ist die chemische Verschiebung als δ-Wert in ppm.

### Beispiel V-1

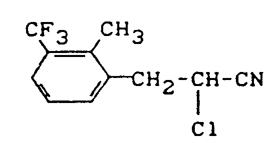

Eine Suspension aus 16,7 g 2-Methyl-3-trifluormethylanilin (0,095 Mol) in 50 ml Wasser wird mit 20 ml konzentrierter Salzsäure bei 0-5°C versetzt und anschließend kurz auf 60-80°C erwärmt, bis sich eine klare Lösung gebildet hat. Man kühlt sehr schnell unter starkem Rühren auf 0-5°C ab, tropft zu der feinen Suspension bei 0-5°C 22,4 ml (0,286 Mol) Acrylnitril und anschließend eine Lösung aus 6,75 g Natriumnitrit in 10 ml Wasser zügig zu, hält die Mischung noch 5 Minuten bei 5°C und läßt 1 Stunde bei 20°C nachrühren. Nach Abkühlen versetzt man die Reaktionsmischung bei 0-10°C portionsweise mit 0,8 g Kupfer-II-oxid-Pulver, wobei eine heftige Stickstoffgasentwicklung eintritt. Nach beendeter Gasentwicklung rührt man noch 2 Stunden bei 0-10°C, anschließend 15 Stunden bei Raumtemperatur, gibt Dichlormethan zu, wäscht mit Wasser, trocknet über Natriumsulfat und engt im Vakuum ein. Man erhält 21,2 g (90% d. Th.) 2-Chlor-3-(2-methyl-3-trifluormethyl-phenyl)propionitril als braunes Öl.
M/e 247 (M⁺), 228, 212, 173 (100%)

### Beispiel V-2

In analoger Weise zu Beispiel (V-1) und gemäß den allgemeinen Angaben zur Herstellung wird 2-Chlor-3-(2,3-Ditrifluormethyl-phenyl)-propionitril erhalten.

M/e 301 (M⁺), 282, 246, 227 (100%), 177.

### Anwendungsbeispiele

In den folgenden Anwendungsbeispielen wurden die nachstehend aufgeführten Verbindungen als Vergleichssubstanzen eingesetzt: 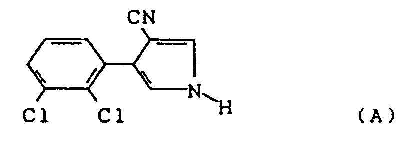
3-Cyano-4-(2,3-dichlorphenyl)-pyrrol
(vgl. EP-A 174 910 sowie EP-A 236 272). 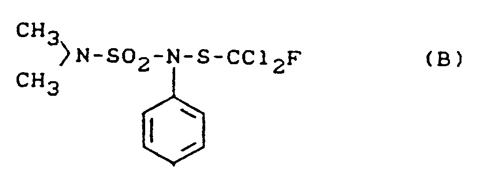
N,N-Dimethyl-N'-phenyl-N'-(fluordichlormethylthio)sulfamid (bekannt aus DE-B 1193498).

### Beispiel A

### Botrytis-Test (Buschbohnen)/protektiv

- Lösungsmittel:: 4,7 Gewichtsteile Aceton
- Emulgator:: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigt in diesem Test z.B. die Verbindung gemäß Herstellungsbeispiel 1.

### Beispiel B

### Pyricularia-Test (Reis)/protektiv

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigt in diesem Test z.B. die Verbindung gemäß Herstellungubeispiel 1.

### Beispiel C

### Erysiphe-Test (Gerste)/protektiv

- Lösungsmittel:: 100 Gewichtsteile Dimethylformamid
- Emulgator:: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigt bei diesem Test z.B. die Verbindung gemäß Herstellungsbeispiel 1.

## Patentansprüche

1. 3-Cyano-4-phenyl-pyrrol-Derivate der Formel (I), 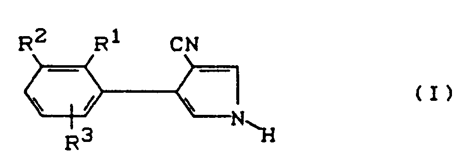 in welcher
R¹ für Chlor, Brom, geradkettiges oder verzweigtes Alkyl mit 1 bis 4 Kohlenstoffatomen oder für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen steht,
R² für Chlor, Brom, geradkettiges oder verzweigtes Alkyl mit 1 bis 4 Kohlenstoffatomen oder für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen steht und
R³ für Wasserstoff oder Fluor steht,
mit der Maßgabe, daß mindestens einer der Reste R¹ oder R² für Halogenalkyl steht.

2. 3-Cyano-4-phenyl-pyrrol-Derivate der Formel (I) gemäß Anspruch 1, bei welchen
R¹ für Chlor, Brom, Methyl, Ethyl, Trifluormethyl oder Trifluormethoxy steht,
R² für Chlor, Brom, Methyl, Ethyl, Trifluormethyl oder Trifluormethoxy steht und
R³ für Wasserstoff oder Fluor steht,
mit der Maßgabe, daß mindestens einer der Reste R¹ oder R² für Trifluormethyl steht.

3. Verfahren zur Herstellung von 3-Cyano-4-phenylpyrrol-Derivaten der Formel (I) gemäß Anspruch 1,
dadurch gekennzeichnet, daß man substituierte Zimtsäurenitrile der Formel (II) 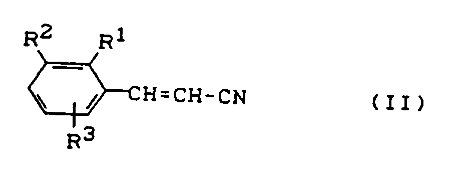 in welcher
R¹, R² und R³ die oben angegebene Bedeutung haben,
mit Sulfonylmethylisocyaniden der Formel (III),
R⁴-SO₂-CH₂-NC (III)
in welcher
R⁴ für Alkyl mit 1 bis 4 Kohlenstoffatomen oder für gegebenenfalls einfach substituiertes Phenyl steht,
in Gegenwart einer Base und gegebenenfalls in Gegenwart eines Verdünnungsmittels umsetzt.

4. Fungizide Mittel, gekennzeichnet durch einen Gehalt an mindestens einem 3-Cyano-4-phenyl pyrrol-Derivat der Formel (I) nach den Ansprüchen 1 und 2.

5. Verwendung von 3-Cyano-4-phenyl-pyrrol-Derivaten der Formel (I) nach den Ansprüchen 1 und 2 zur Bekämpfung von Pilzen.

6. Verfahren zur Bekämpfung von Pilzen in Pflanzen, dadurch gekennzeichnet, daß man mindestens ein 3-Cyano-4-Phenyl-pyrrol-Derivat der Formel (I) nach den Ansprüchen 1 und 2 auf Pilze und/oder ihren Lebensraum einwirken läßt.

7. Verfahren zur Herstellung von fungiziden Mitteln, dadurch gekennzeichnet, daß man 3-Cyano-4-phenyl-pyrrol-Derivate der Formel (I) nach den Ansprüchen 1 und 2 mit Streckmitteln und/oder oberflächenaktiven Mitteln vermischt.

8. Substituierte Zimtsäurenitrile der Formel (II) 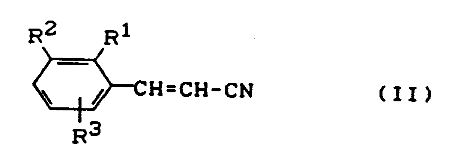 in welcher
R¹, R² und R³ die im Anspruch 1 angegebenen Bedeutungen haben.

9. Verfahren zur Herstellung von substituierten Zimtsäurenitrilen der Formel (II) 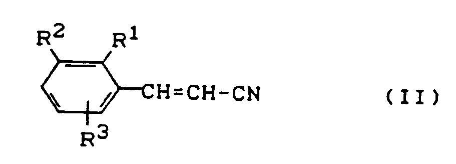 in welcher
R¹, R² und R³ die im Anspruch 1 angegebenen Bedeutungen haben,
dadurch gekennzeichnet, daß man
a) Aniline der Formel (IV), 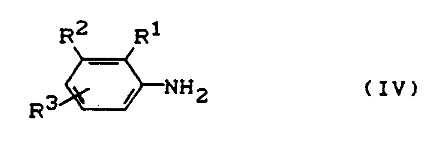 in welcher
R¹, R² und R³ die oben angegebene Bedeutung haben,
zunächst in einer ersten Stufe mit Acrylnitril in Gegenwart von Natriumnitrit und Salzäure in Gegenwart eines geeigneten Metallsalz-Katalysators, gegebenenfalls in Gegenwart eines geeigneten Verdünnungsmittels bei Temperaturen zwischen -20 °C und +50 °C umsetzt und dann in einer 2. Stufe die so erhältlichen substituierten α-Chlor-β-phenylpropionitrile der Formel (V), 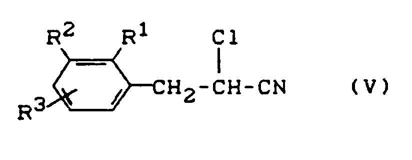 in welcher
R¹, R² und R³ die oben angegebene Bedeutung haben,
mit Basen, gegebenenfalls in Gegenwart eines Verdünnungsmittels bei Temperaturen zwischen 0 °C und 50 °C dehydrohalogeniert oder
b) Benzaldehyde der Formel (VI), 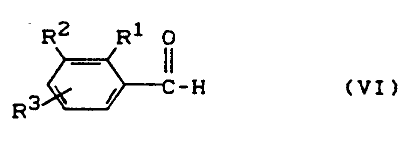 in welcher
R¹, R² und R³ die oben angegebene Bedeutung haben,
mit Cyanessigsäure der Formel (VII),
NC-CH₂-COOH (VII)
in Gegenwart einer Base und gegebenenfalls in Gegenwart eines geeigneten Verdünnungsmittels, bei Temperaturen zwischen 50 °C und 120 °C kondensiert und gleichzeitig decarboxyliert.

## Claims

1. 3-Cyano-4-phenyl-pyrrole derivatives of the formula (I) 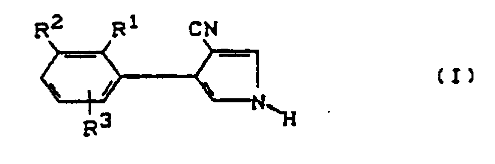 in which
R¹ represents chlorine, bromine, straight-chain or branched alkyl having 1 to 4 carbon atoms or in each case straight-chain or branched halogenoalkyl or halogenoalkoxy, each of which has 1 to 4 carbon atoms and 1 to 9 identical or different halogen atoms,
R² represents chlorine, bromine, straight-chain or branched alkyl having 1 to 4 carbon atoms or in each case straight-chain or branched halogenoalkyl or halogenoalkoky, each of which has 1 to 4 carbon atoms and 1 to 9 identical or different halogen atoms, and
R³ represents hydrogen or fluorine,
with the proviso that at least one of the radicals R¹ or R² represents halogenoalkyl.

2. 3-Cyano-4-phenyl-pyrrole derivatives of the formula (I) according to Claim 1, in which
R¹ represents chlorine, bromine, methyl, ethyl, trifluoromethyl or trifluoromethoxy,
R² represents chlorine, bromine, methyl, ethyl, trifluoromethyl or trifluoromethoxy, and
R³ represents hydrogen or fluorine,
with the proviso that at least one of the radicals R¹ or R² represents trifluoromethyl.

3. Process for the preparation of 3-cyano-4-phenylpyrrole derivatives of the formula (I) according to Claim 1,
characterised in that substituted cinnamonitriles of the formula (II) 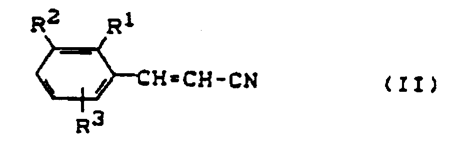 in which
R¹, R² and R³ have the abovementioned meaning,
are reacted with sulphonylmethyl isocyanides of the formula (III)
R⁴-SO₂-CH₂-NC (III)
in which
R⁴ represents alkyl having 1 to 4 carbon atoms or optionally monosubstituted phenyl,
in the presence of a base and, if appropriate, in the presence of a diluent.

4. Fungicidal agents, characterised in that they contain at least one 3-cyano-4-phenyl-pyrrole derivative of the formula (I) according to Claims 1 and 2.

5. Use of 3-cyano-4-phenyl-pyrrole derivatives of the formula (I) according to Claims 1 and 2 for combating fungi.

6. Method of combating fungi in plants, characterised in that at least one 3-cyano-4-phenyl-pyrrole derivative of the formula (I) according to Claims 1 and 2 are allowed to act on fungi and/or their environment.

7. Process for the preparation of fungicidal agents, characterised in that 3-cyano-4-phenyl-pyrrole derivatives of the formula (I) according to Claims 1 and 2 are mixed with extenders and/or surface-active agents.

8. Substituted cinnamonitriles of the formula (II) 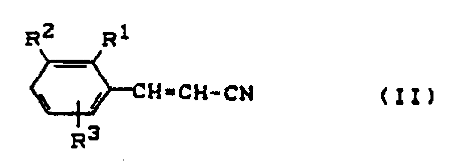 in which
R¹, R² and R³ have the meanings given in Claim 1.

9. Process for the preparation of substituted cinnamonitriles of the formula (II) 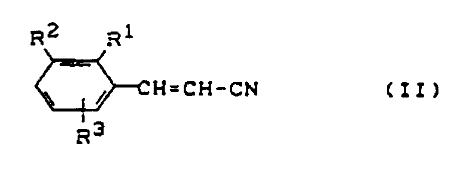 in which
R¹, R² and R³ have the meanings given in Claim 1,
characterised in that
a) anilines of the formula (IV) 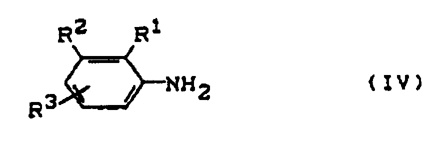 in which
R¹, R² and R³ have the abovementioned meaning,
are first reacted, in a first step, with acrylonitrile in the presence of sodium nitrite and hydrochloric acid in the presence of a suitable metal salt catalyst, if appropriate in the presence of a suitable diluent, at temperatures between -20°C and +50°C, and then, in a 2nd step, the resulting substituted α-chloro-β-phenylpropionitriles of the formula (V) 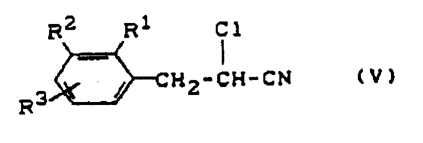 in which
R¹, R² and R³ have the abovementioned meaning,
are dehydrohalogenated with bases, if appropriate in the presence of a diluent, at temperatures between 0°C and 50°C, or
b) benzaldehydes of the formula (VI) 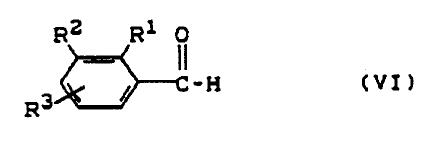 in which
R¹, R² and R³ have the abovementioned meaning,
are subjected to a condensation reaction with cyanoacetic acid of the formula (VII),
NC-CH₂-COOH (VII)
in the presence of a base and, if appropriate, in the presence of a suitable diluent, at temperatures between 50°C and 120°C, and, at the same time, decarboxylated.

## Revendications

1. Dérivés de 3-cyano-4-phényl-pyrrole de formule (I), 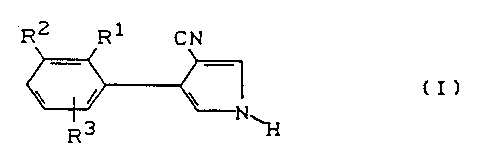 dans laquelle
R¹ représente le chlore, le brome, un groupe alkyle à chaîne droite ou ramifiée ayant 1 à 4 atomes de carbone ou un groupe halogénalkyle ou halogénalkoxy chacun à chaîne droite ou ramifiée, avec chacun 1 à 4 atomes de carbone et 1 à 9 atomes d'halogènes identiques ou différents,
R² représente le chlore, le brome, un groupe alkyle à chaîne droite ou ramifiée ayant 1 à 4 atomes de carbone ou un groupe halogénalkyle ou halogénalkoxy chacun à chaîne droite ou ramifiée, avec chacun 1 à 4 atomes de carbone et 1 à 9 atomes d'halogènes identiques ou différents et
R³ représente l'hydrogène ou le fluor,
sous réserve que l'un au moins des restes R¹ ou R² représente un reste halogénalkyle.

2. Dérivés de 3-cyano-4-phényl-pyrrole de formule (I) suivant la revendication 1, dans lesquels
R¹ représente le chlore, le brome, un groupe méthyle, éthyle, trifluorométhyle ou trifluorométhoxy,
R² représente le chlore, le brome, un groupe méthyle, éthyle, trifluorométhyle ou trifluorométhoxy et
R³ est l'hydrogène ou le fluor,
sous réserve que l'un au moins des restes R¹ ou R² soit un reste trifluorométhyle.

3. Procédé de production de dérivés de 3-cyano-4-phényl-pyrrole de formule (I) suivant la revendication 1, caractérisé en ce qu'on fait réagir des nitriles d'acide cinnamique substitués de formule (II) 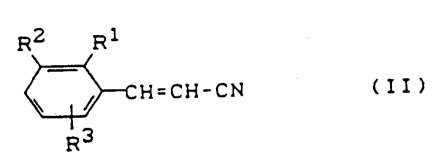 dans laquelle
R¹, R² et R³ ont la définition indiquée ci-dessus,
avec des sulfonylméthylisocyanures de formule (III),
R⁴-SO₂-CH₂-NC (III)
dans laquelle
R⁴ est un groupe alkyle ayant 1 à 4 atomes de carbone ou un groupe phényle portant éventuellement un substituant,
en présence d'une base et, le cas échéant en présence d'un diluant.

4. Compositions fongicides, caractérisées par une teneur en au moins un dérivé de 3-cyano-4-phényl-pyrrole de formule (I) suivant les revendications 1 et 2.

5. Utilisation de dérivés de 3-cyano-4-phényl-pyrrole de formule (I) suivant les revendications 1 et 2 pour combattre des champignons.

6. Procédé pour combattre des champignons attaquant des plantes, caractérisé en ce qu'on fait agir au moins un dérivé de 3-cyano-4-phényl-pyrrole de formule (I) suivant les revendications 1 et 2 sur les champignons et/ou sur leur milieu.

7. Procédé de préparation de compositions fongicides, caractérisé en ce qu'on mélange des dérivés de 3-cyano-4-phényl-pyrrole de formule (I) suivant les revendications 1 et 2 avec des diluants et/ou des agents tensio-actifs.

8. Nitriles d'acide cinnamique substitués de formule (II) 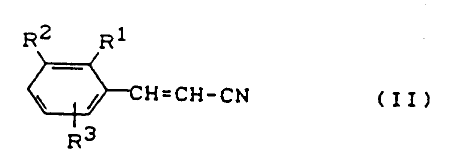 dans laquelle
R¹, R² et R³ ont les définitions indiquées dans la revendication 1.

9. Procédé de production de nitriles d'acide cinnamique substitués de formule (II) 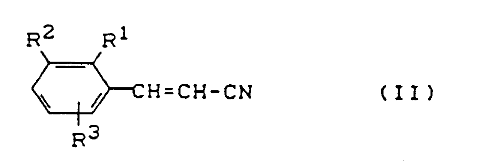 dans laquelle
R¹, R² et R³ ont les définitions indiquées dans la revendication 1,
caractérisé en ce que
a) on fait réagir des anilines de formule (IV), 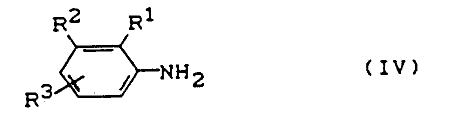 dans laquelle
R¹, R² et R³ ont la définition indiquée ci-dessus,
tout d'abord dans une première étape avec l'acrylonitrile en présence de nitrite de sodium et d'acide chlorhydrique, en présence d'un catalyseur formé d'un sel métallique approprié, éventuellement en présence d'un diluant approprié, à des températures comprises entre -20°C et +50°C, puis on déshydrohalogène dans une seconde étape les α-chloro-β-phénylpropionitriles substitués ainsi obtenus de formule (V), 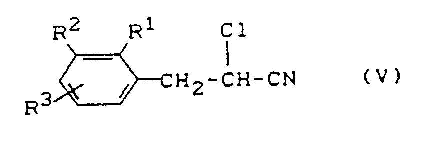 dans laquelle
R¹, R² et R³ ont la définition indiquée ci-dessus,
avec des bases, le cas échéant en présence d'un diluant, à des températures comprises entre 0°C et 50°C, ou bien
b) on condense des benzaldéhydes de formule (VI), 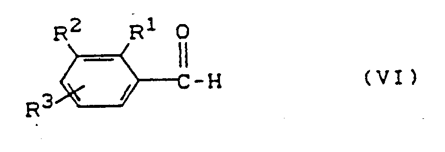 dans laquelle
R¹, R² et R³ ont la définition indiquée ci-dessus,
avec l'acide cyanacétique de formule (VII),
NC-CH₂-COOH (VII)
en présence d'une base et en la présence éventuelle d'un diluant approprié, à des températures comprises entre 50°C et 120°C et on effectue en même temps une décarboxylation.
